(19) Europäisches Patentamt
European Patent Office
Office européen des brevets

(11) Veröffentlichungsnummer: **0 158 144**
**B1**

# (12) EUROPÄISCHE PATENTSCHRIFT

(45) Veröffentlichungstag der Patentschrift: **20.04.88**

(51) Int. Cl.⁴: **C 07 D 233/58**, C 07 D 233/60, A 61 K 31/415

(21) Anmeldenummer: **85102881.1**

(22) Anmeldetag: **13.03.85**

**E R R A T U M**

| DIE TEXTSTELLE:<br>TEXT PUBLISHED:<br>LE PASSAGE SUIVANT: | | (SEITE, SPALTE, ZEILE)<br>(PAGE, COLUMN, LINE)<br>(PAGE, COLONNE, LIGNE) | | LAUTET BERICHTIGT:<br>SHOULD READ:<br>DEVRAIT ETRE LU: |
|---|---|---|---|---|
| 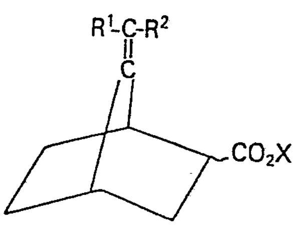 VI | 3 | 3 | 58/65 | 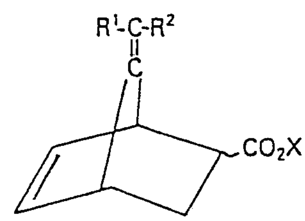 VI |
| worin n O oder A | 3 | 4 | 22 | worin n O und A |
| in Formel = B die | 11 | 19 | 2 | in Formel I B die |
| N-Bicycloheptenyl-imidazole der | 11 | 20 | 20 | N-Bicycloheptenyl-imidazolen der |
| sowie deren physiologisch | 11 | 20 | 51 | sowie von deren physiologisch |
| n-bicycloheptenyl-imidazoles | 13 | 24 | 4 | N-bicycloheptenyl-imidazoles |

Tag der Entscheidung über die Berichtigung / Date of decision on rectification: / Date de décision portant sur modification: 19.9.88

Ausgabe- und Veröffentlichungstag: / Issue and publication date: / Date d'edition et de publication: 30.11.88

Patbl.Nr) / EPB no:) / Bull. no:) 88/48

(19) Europäisches Patentamt
European Patent Office
Office européen des brevets

(11) Veröffentlichungsnummer: **0 158 144**
**B1**

(12) # EUROPÄISCHE PATENTSCHRIFT

(45) Veröffentlichungstag der Patentschrift: **20.04.88**

(51) Int. Cl.4: **C 07 D 233/58**, C 07 D 233/60, A 61 K 31/415

(21) Anmeldenummer: **85102881.1**

(22) Anmeldetag: **13.03.85**

(54) **N-Bicycloheptyl- und N-Bicycloheptenyl-imidazole, Verfahren zu ihrer Herstellung, ihre Verwendung sowie pharmazeutische Präparate.**

(30) Priorität: **22.03.84 DE 3410498**

(43) Veröffentlichungstag der Anmeldung: **16.10.85 Patentblatt 85/42**

(45) Bekanntmachung des Hinweises auf die Patenterteilung: **20.04.88 Patentblatt 88/16**

(84) Benannte Vertragsstaaten: **AT BE CH DE FR GB IT LI LU NL SE**

(56) Entgegenhaltungen:
**EP - A - 0 064 245**
**US - A - 3 682 951**

(73) Patentinhaber: **HOECHST AKTIENGESELLSCHAFT, Postfach 80 03 20, D-6230 Frankfurt am Main 80 (DE)**

(72) Erfinder: **Siegel, Herbert, Dr., Am Schieferberg 10, D-6233 Kelkheim (Taunus) (DE)**
Erfinder: **Granzer, Ernold, Dr., Falkensteiner Strasse 24, D-6233 Kelkheim (Taunus) (DE)**

**EP 0 158 144 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft N-Bicycloheptyl- und N-Bicycloheptenyl-imidazole, Verfahren zu ihrer Herstellung, diese Verbindungen enthalten pharmazeutische Präparate sowie ihre Verwendung als Arzneimittel, insbesondere zur Behandlung von Hyperlipidämien.

Es wurde bereits beschrieben, dass 1-(Cycloalkyl)alkyl-imidazole spermizide Wirkung haben (vgl. europäische Patentanmeldung mit der Veröffentlichungs-Nr. 0064245). Entsprechend den Angaben der US-Patentschrift 3682951 sind die dort beschriebenen 1-[β-(1-Adamantyloxy)halogenphenylethyl]imidazole wirksam gegen Bakterien. Ferner wurde beschrieben, dass Imidazole, welche am Stickstoffatom mit einem geradkettigen oder verzweigten Alkylrest oder mit dem Benzylrest substituiert sind, eine hypolipidämische Wirkung aufweisen [vgl. J. Med. Chem. 18, 833 (1975)]. Um therapeutisch befriedigende Resultate zu erzielen, sind jedoch hohe Dosen der dort untersuchten Verbindungen erforderlich. In der deutschen Offenlegungsschrift 2944663 werden imidazolmethylsubstituierte Bicyclen mit antithromboembolischer Wirkung beschrieben. Sofern es sich bei den dort erwähnten Bicyclen um Bicycloheptane handelt, sind sie in 7-Stellung unsubstituiert.

Es wurde überraschenderweise gefunden, dass in 7-Stellung substituierte N-Bicycloheptyl- und N-Bicycloheptenylimidazole eine viel höhere hypolipidämische Wirkung aufweisen als bisher beschriebene Imidazolderivate.

Die Erfindung betrifft daher neue N-Bicycloheptyl- und N-Bicycloheptenyl-imidazole der Formel I

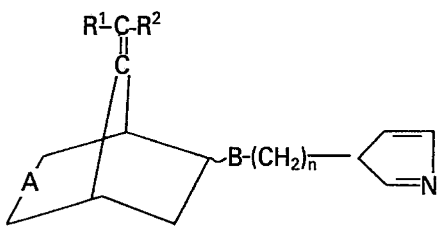

I

worin

$R^1$ und $R^2$ gleich oder verschieden sind und Alkyl mit 1 bis 10 C-Atomen, Cycloalkyl mit 4 bis 10 C-Atomen, Phenyl, das gegebenenfalls mono- oder disubstituiert ist mit Halogen, $(C_1–C_4)$-Alkyl, $(C_1–C_4)$-Alkoxy, Trifluormethyl, Hydroxy, Amino $(C_1–C_4)$-Alkylamino oder $(C_1–C_4)$-Dialkylamino, wobei im Falle der Disubstitution die Substituenten gleich oder verschieden sind, Naphthyl oder Phenylalkyl mit 1 bis 4 Alkylkohlenstoffatomen bedeuten, oder $R^1$ und $R^2$ gemeinsam für eine gegebenenfalls mit Phenyl substituierte $(CH_2)_m$-Brücke, wobei m eine Zahl von 3 bis 10 bedeutet, stehen,

A eine Einfach- oder Doppelbindung darstellt,

B eine einfache Bindung, oder, falls n für 0 oder 1 steht, auch die $\underset{\underset{O}{\|}}{-C-}$-Gruppe, oder, falls n für 1 steht, die -CHOH-Gruppe bedeutet und

n für 0, 1, 2, 3 oder 4 steht,

sowie deren physiologisch verträgliche Säureadditionssalze.

Unter Alkyl wird sowohl geradkettiges als auch verzweigtes Alkyl verstanden.

Die Schlangenlinie in Formel I sowie in den folgenden Formeln besagt, dass die Substituenten sowohl in der endo- als auch in der exo-Stellung am Bicyclus stehen können.

Bevorzugt sind Verbindungen der Formel I, worin $R^1$ und $R^2$ gleich oder verschieden sind und Phenyl sowie mit Fluor oder Chlor in 4-Stellung monosubstituiertes Phenyl bedeuten. A stellt vorzugsweise eine einfache Bindung dar, n bedeutet insbesondere 0 oder 1 und B eine Einfachbindung bzw. die -CO-Gruppe.

Das Verfahren zur Herstellung der Verbindungen der Formel I und deren Salze ist dadurch gekennzeichnet, dass man

a) einen Alkohol der Formel II

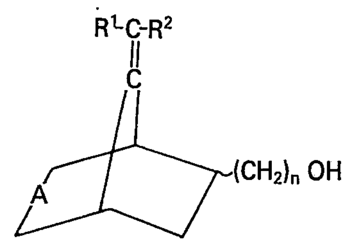

II

worin A, $R^1$, $R^2$ und n die zu Formel I angegebenen Bedeutungen haben, nach an sich bekannten Methoden in eine Verbindung der Formel III

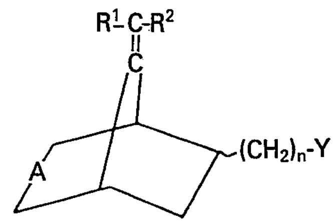

III

worin A, $R^1$, $R^2$ und n die zu Formel I angegebenen Bedeutungen haben und Y eine leaving group wie z. B. Halogen, insbesondere Chlor oder Brom, oder ein Sulfonsäurederivat, wie

$(C_1–C_4)$-Alkyl-$SO_2$-O-, 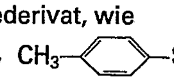 oder

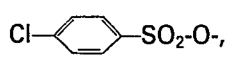, bedeutet, überführt und mit Imidazol oder einem Imidazolsalz der Formel IV

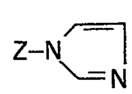

IV

worin Z Wasserstoff oder Alkalimetall bedeutet zu einer Verbindung der Formel I, worin $R^1$, $R^2$, A und n die angegebenen Bedeutungen haben und B eine einfache Bindung darstellt, umsetzt, und gegebenenfalls in einer erhaltenen Verbindung, worin A eine Doppelbindung darstellt, die Doppelbindung hydriert, wobei die Hydrierung entweder an einem Zwischenprodukt oder an einer Verbindung der Formel I (A = Doppelbindung) durchgeführt

wird, oder

b) ein Halogenid der Formel V

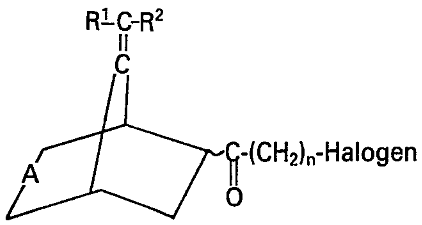

V

worin A, $R^1$ und $R^2$ die zu Formel I angegebenen Bedeutungen haben, n für 0 oder 1 steht und Halogen für Brom oder Chlor steht, mit Imidazol oder einem Imidazolsalz der Formel IV zu einer Verbindung der Formel I, worin A, $R^1$ und $R^2$ die angegebenen Bedeutungen haben, B die $\underset{\underset{O}{\|}}{C}$-Gruppe, und n = 0 oder 1 sind, umsetzt und gegebenenfalls zu einer Verbindung, worin B die CHOH-Gruppe bedeutet, reduziert und gewünschtenfalls die nach a) oder b) erhaltenen Verbindungen mit einer Säure in die physiologisch verträglichen Säureadditionssalze überführt.

Die Alkohole der Formel II werden gemäss Verfahren a) beispielsweise mit einem anorganischen Halogenid, wie z. B. $POCl_3$, $PCl_3$, $PBr_3$, $P(C_6H_5)_3/CCl_4$ oder $SOCl_2$, in die Halogenverbindung III (Y = Halogen) umgewandelt oder man überführt sie mit einem Sulfonsäurehalogenid in den Sulfonsäureester. Als Sulfonsäurehalogenide kommen die gebräuchlichen Halogenide wie Mesylchlorid, Tosylchlorid, p-Chlorbenzolsulfochlorid in Frage. Die Substitution am Halogenid oder Sulfonsäureester erfolgt zweckmässigerweise mit Imidazol in Gegenwart eines säurebindenden Zusatzstoffes wie einem aliphatischen oder aromatischen Amin oder mit dem Natriumsalz von Imidazol in einem polaren Lösungsmittel wie DMF, DMSO, THF, Alkohol bei Temperaturen zwischen 0 und 100 °C.

Die Alkohole der Formel II sind entweder in der Literatur beschrieben oder werden analog beschriebenen Methoden hergestellt. Bei der Herstellung von Alkoholen der Formel II worin n gleich 1 ist, geht man zweckmässigerweise wie folgt vor:

Entsprechend der in Ann. 566, Seiten 1ff und 27ff (1950) angegebenen Methode erhält man aus $R^1$, $R^2$-substituierten Pentafulvenen und entsprechend substituierten Olefinen wie z. B. Acrylsäure, Acrylsäureestern, Acrylnitril, α-Chloracrylnitril, Methylvinylketon und anderen elektronenarmen Olefinen ohne Lösungsmittel oder in Gegenwart eines Lösungsmittels bei Temperaturen von zweckmässigerweise 20–80 °C Bicycloheptenderivate. Das Bicycloheptenderivat der Formel VI

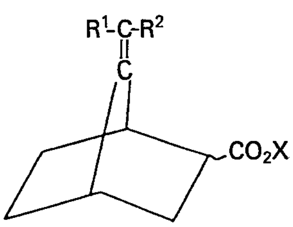

VI

worin $R^1$ und $R^2$ die angegebenen Bedeutungen haben und X Wasserstoff oder eine $(C_1–C_4)$-Alkylgruppe bedeutet, erhält man durch Umsetzung mit Acrylsäure oder einem entsprechenden Acrylsäureester. Die Umsetzung ist vielfach rascher als in Ann. 566, 1ff. angegeben. Die Reaktionsdauer liegt meist zwischen wenigen Stunden und einigen Tagen.

Zum Aufbau des Bicyclus verwendeten Fulvene werden nach literaturbekannten Methoden erhalten [vgl. Adv. in Alicyclic Chem. 2, 59 (1968)].

Besonders geeignet für Verfahrensweise a) sind die Verbindungen der Formel VI. Sie werden mit einem Reduktionsmittel, z. B. einem komplexen Hydrid wie $LiAlH_4$, in den entsprechenden Alkohol überführt.

Zu einem Alkohol der Formel II, worin n 1 ist und A eine Einfachbindung darstellt, gelangt man durch Hydrierung nach an sich bekannten Methoden.

Bei der Herstellung der Alkohole der Formel II, worin n O oder A eine Einfachbindung darstellen, geht man z. B. von einem Keton der Formel VII

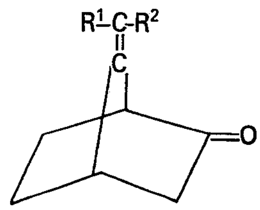

VII

aus und reduziert es z. B. mit $LiAlH_4$ zum gewünschten Alkohol.

Durch Umsetzung des gleichen Ketons VII mit metallorganischen Reagenzien wie $(EtO)_2POCH$-NaCOX oder einem Wittig-Reagenz wie beispielsweise $(C_6H_5)_3P=CH-(CH_2)_{n-2}-COX$ gelangt man zu Verbindungen der Formel VIII

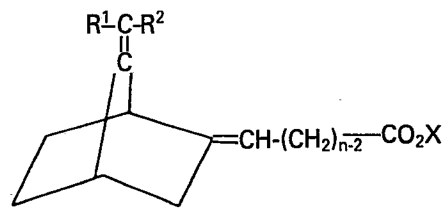

VIII

worin X das Kation eines Alkalimetalls oder $(C_1–C_4)$-Alkyl bedeutet. Die Reduktion zum Alkohol erfolgt, z. B. wie oben angegeben. Bei der anschliessenden Hydrierung wird nur die Doppelbindung in der Seitenkette hydriert.

Die Hydrierung der endocyclischen Doppelbindung (A = Doppelbindung) ist im Prinzip auf jeder Stufe der Synthesefolge möglich. Sie wird jedoch bevorzugt auf der Stufe des Cycloadduktes (Verbindung der Formel VI), oder am Imidazolderivat I selbst durchgeführt. Die Verfahrensweise ist an sich bekannt, als Katalysator dient z. B. 5% Pd auf Kohle. Man gelangt so zu den erfindungsgemässen Verbindungen mit A = Einfachbindung.

Die Herstellung von Verbindungen der Formel I, die eine Keto- oder Hydroxygruppe in der Imidazol-substituierten Seitenkette enthalten (B = CO oder CHOH) erfolgt gemäss Verfahren b. Das als Ausgangsmaterial verwendete Halogenid der For-

mel V erhält man nach literaturbekannten Methoden. Das in den Ann. 566, 27 (1950) beschriebene Methylketon, das vorzugsweise eingesetzt wird, wurde unter kinetischer Kontrolle in den Silylenolether überführt [vgl. JOC 34, 2324 (1969)] und dieser dann bromiert (vgl. Synthesis 1976, 194).

Zu dem erfindungsgemässen Imidazolderivat gelangt man, indem man das entsprechende Halogenid vorzugsweise in einem polaren organischen Lösungsmittel wie DMF, DMSO, THF, niedermolekularen Alkoholen bei einer Temperatur zwischen 0 und 100 °C, vorzugsweise Raumtemperatur, mit der mindestens doppelten Menge Imidazol oder dem Na-Salz von Imidazol umsetzt. Das erhaltene Ketoimidazolderivat (B = CO) kann gegebenenfalls zum entsprechenden Alkohol (B = CHOH) reduziert werden. Zur Reduktion eignen sich am besten komplexe Hydride, wie z. B. $LiAlH_4$ oder $NaBH_4$ in einem geeigneten Lösungsmittel.

Aus den meist als Öl anfallenden Imidazolderivaten der allgemeinen Formel (I) können Säureadditionssalze hergestellt werden. Dazu kommen alle Säuren, die physiologisch verträglichen Salze bilden, in Frage. Dazu gehören sowohl anorganische Säuren, wie z. B. Chlorwasserstoffsäure, Salpetersäure und Schwefelsäure als auch mono- und bifunktionelle organische Säuren, insbesondere Carbonsäuren wie Essigsäure, Bernsteinsäure, Weinsäure usw.

Die erfindungsgemässen Verbindungen der Formel I besitzen wertvolle pharmakologische Eigenschaften, insbesondere zeigen sie eine sehr starke und günstige Beeinflussung der Serumlipoproteine. Die Erfindung betrifft daher auch pharmazeutische Präparate auf Basis dieser Verbindungen sowie ihre Verwendung als Arzneimittel, insbesondere zur Beeinflussung der Serumlipoproteine.

Es ist allgemein anerkannt, dass für die Entstehung arteriosklerotischer Gefässveränderungen, insbesondere der coronaren Herzkrankheit, Hyperlipoproteinämien einen wesentlichen Risikofaktor darstellen. Für die Prophylaxe und die Regression von arteriosklerotischen Veränderungen kommt daher die Senkung erhöhter Serum-Lipoproteine eine ausserordentliche Bedeutung zu. Hierbei kommt es aber auf ganz bestimmte Klassen von Serum-Lipoproteinen an, da die low density (LDL) und very low density-Lopoproteine (VLDL) einen atherogenen Risikofaktor darstellen, während die high density-Lipoproteine (HDL) eine Schutzfunktion gegenüber arteriosklerotischer Gefässveränderung ausüben. Hypolipidämika sollen demnach VLDL-Cholesterin und LDL-Cholesterin im Serum erniedrigen, dabei aber die HDL-Cholesterin-Konzentration nach Möglichkeit unbeeinflusst lassen oder sogar erhöhen, so dass der

antiatherogene Index $\frac{HDL}{LDL}$ zunimmt.

Die erfindungsgemässen Verbindungen haben wertvolle therapeutische Eigenschaften. So erniedrigen sie vor allem die Konzentration von LDL und VLDL, die HDL-Fraktion aber nur in überhöhter Dosierung, die bereits die LDL-Cholesterinkonzentration um mehr als ca. 50% vermindern, so dass im therapeutisch nutzbaren Bereich eine starke Verminderung der LDL-Fraktion ohne Beeinflussung der HDL-Fraktion resultiert. Diese Verbindungen stellen daher einen wesentlichen Fortschritt gegenüber der Vergleichsverbindung Clofibrat dar, das neben LDL immer eine sehr starke Verminderung der HDL bewirkt, wie aus den nachfolgend beschriebenen Daten ersichtlich ist. Sie können daher zur Prophylaxe und Regression von arteriosklerotischen Veränderungen herangezogen werden, indem sie einen kausalen Risikofaktor ausschalten.

Hierzu zählen nicht nur die primären Hyperlipidämien, sondern auch gewisse sekundäre Hyperlipidämien wie sie z. B. beim Diabetes vorkommen. Das relative Lebergewicht wird durch die Verbindungen in wirksamer Dosis nicht oder nur geringfügig vermehrt, während das als hypolipidämischer Standard verwendete Clofibrat zu einer starken Erhöhung des relativen Lebergewichtes führt.

Die Wirkung der angeführten Verbindungen auf die Serum-Lipoproteine wurde an männlichen Wistar-Ratten untersucht, die 7 Tage per Schlundsonde mit den angeführten in Polyäthylenglykol 400 suspendierten Verbindungen behandelt wurden. Ausserdem wurde eine Kontrollgruppe, die nur das Lösungsmittel Polyäthylenglykol 400 erhielt, mitgeführt, sowie bei den meisten Versuchen eine Ratten-Gruppe mit dem Standard-Hypolipidämikum Clofibrat. Pro Gruppe wurden in der Regel 10 Tiere eingesetzt, denen am Ende der Behandlung das Blut nach leichter Äthernarkose aus dem Orbitalplexus entnommen wurde. Aus dem gewonnenen Ratten-Serum wurden die Serum-Lipoproteine in der präparativen Ultrazentrifuge in folgende Dichteklassen getrennt:

VLDL < 1.006; LDL 1.006 bis 1.04; HDL 1,04 bis 1,21

Da im Gegensatz zum Menschen die Serumlipoproteine der Ratte etwa 4/5 HDL-Cholesterin und nur 1/5 LDL-Cholesterin enthalten und nur sehr geringe Mengen von VLDL (beim Menschen umgekehrt etwa 4/5 LDL und VLDL und nur 1/5 HDL), setzt die Beurteilung einer hypolipidämischen Wirkung an der Ratte unbedingt die Fraktionierung des Rattenserums in Lipoproteinklassen voraus. Eine blosse Erniedrigung des Serum-Total-Cholesteringehaltes an der Ratte würde nämlich nur die unerwünschte Erniedrigung der bei der Ratte vorwiegenden antiatherogenen HDL-Klasse anzeigen. Eine erwünschte Erniedrigung von LDL bei gleichzeitiger erwünschter Vermehrung von HDL hätte aber keine (wesentliche) Auswirkung auf den Totalcholesteringehalt des Ratten-Serums.

Aus den in der Ultrazentrifuge isolierten Lipoproteinfraktionen wurde das darin enthaltene Cholesterin vollenzymitisch nach der CHOD-PAP-Methode mittels der Testkombination von Boehringer-Mannheim bestimmt und die Werte in µg/ml Serum umgerechnet. In der angeführten Tabelle I ist die prozentuale Veränderung des Lipoprotein-Cholesterins in der behandelten Gruppe gegenüber einer unter gleichen Bedingungen mitgeführten Kontrollgruppe angegeben. Wie aus der Tabelle I ersichtlich, bewirkt Clofibrat eine stärkere Verminderung der HDL- als der LDL-Fraktion, während die neuen Verbindungen eine starke selektiv senkende Wirkung auf die atherogenen Lipoproteinfraktionen (VLDL und LDL) ausüben und die schützende HDL-Fraktion im wesentlichen unbeeinflusst lassen.

Tabelle I
Veränderungen der Lipoproteine im Ratten-Serum in % nach 7tägiger oraler Application der Verbindungen

| Beispiel Nr. | Dosierung mg/kg/Tag | Cholesterolveränderung in %, bezogen auf die Kontrollgruppen<br>im Serum | in den Serum-Lipoprotein-Fraktionen<br>VLDL | LDL | HDL | HDL | % Veränderungen des relativen Lebergewichtes |
|---|---|---|---|---|---|---|---|
| 17 | 30 | −55 | | − 88 | −54 | 3,75 | +20 |
| | 12,5 | −47 | | − 82 | −39 | 3,34 | +10 |
| | 5 | −26 | | − 43 | −17 | 1,46 | + 3 |
| | 3 | + 2 | | − 26 | + 2 | 1,38 | + 4 |
| | 1 | +13 | −60 | − 23 | −11 | 1,16 | |
| | 0,3 | + 6 | +13 | − 26 | − 2 | 1,33 | − 1 |
| 15 | 30 | −78 | −74 | − 94 | −95 | 0,93 | +31 |
| | 10 | −96 | −88 | −100 | −98 | | +24 |
| | 3 | −93 | −63 | − 89 | −96 | 0,35 | +24 |
| | 1 | −80 | 0 | − 89 | −83 | 1,49 | +19 |
| | 0,3 | −53 | +19 | − 69 | −58 | 1,37 | + 6 |
| | 0,1 | − 2 | −20 | − 36 | − 9 | 1,43 | + 1 |
| | 0,03 | + 1 | −33 | − 21 | − 5 | 1,78 | − 1 |
| 7 | 3 | −11 | −18 | − 30 | − 4 | 1,36 | − 1 |
| 2 | 30 | + 7 | −31 | − 37 | +11 | 1,76 | +15 |
| 22 | 10 | − 5 | −57 | − 44 | + 4 | 1,85 | +10 |
| | 3 | − 5 | −57 | − 29 | 0 | 1,42 | 0 |
| 4 | 10 | −69 | +50 | − 88 | −74 | 2,13 | +10 |
| | 3 | −36 | − 5 | − 53 | −36 | 1,36 | 0 |
| | 1 | −15 | + 6 | − 36 | −19 | 1,0 | + 1 |
| 20 | 3 | −46 | 0 | −75 | −50 | 2,6 | + 2 |
| | 0,3 | − 2 | − 9 | − 30 | − 9 | 1,30 | − 4 |
| 21 | 0,3 | −18 | −20 | − 25 | −16 | 1,12 | 0 |
| 9 | 30 | −12 | −43 | − 40 | −14 | 1,43 | +10 |
| 14 | 3 | −14 | +32 | − 44 | − 9 | 0,97 | 0 |
| 12 | 0,3 | −15 | −10 | − 25 | −14 | 1,16 | 0 |
| 13 | 0,3 | −17 | −10 | − 37 | −17 | 1,33 | − 2 |
| 24 | 0,1 | −49 | − 6 | − 68 | −40 | 1,84 | +12 |
| 25 | 3 | −16 | −17 | − 36 | −11 | 1,39 | + 2 |
| | 0,3 | − 2 | +10 | − 16 | + 3 | 1,23 | + 8 |
| Clofibrat | 100 | −26 | −10 | − 38 | −23 | 1,18 | +29 |

Als therapeutische Zubereitung der Verbindungen der Formel I kommen vor allem Tabletten, Dragées, Kapseln, Suppositorien und Säfte in Frage. Die neuen Verbindungen können dabei entweder allein oder mit pharmakologisch annehmbaren Trägern vermischt, angewandt werden. Eine orale Anwendungsform wird bevorzugt. Zu diesem Zweck werden die aktiven Verbindungen vorzugsweise mit an sich bekannten Hilfsstoffen vermischt und durch an sich bekannte Methoden in geeignete Darreichungsformen gebracht, wie Tabletten, Steckkapseln, wässrige oder ölige Suspensionen oder wässrige oder ölige Lösungen. Als inerte Träger können z. B. Magnesiumkarbonat, Milchzucker oder Maisstärke unter Zusatz anderer Stoffe, wie z. B. Magnesiumstearat verwendet werden. Dabei kann die Zubereitung als Trocken- oder als Feuchtgranulat erfolgen. Als ölige Trägerstoffe oder Lösungsmittel kommen besonders pflanzliche und tierische Öle in Betracht, wie z. B. Sonnenblumenöl oder Lebertran. Als tägliche Dosis kommen etwa 20 mg bis 1 g, vorzugsweise 50 bis 100 mg in Betracht. Eine Dosierungseinheit enthält vorzugsweise 10 bis 25 mg.

Die Zubereitungen können bei der Behandlung von Lipidstoffwechselstörungen ausser den üblichen Füll- und Trägerstoffen noch ein Antihypertensivum, wie beispielsweise ein Saluretikum, Reserpin, Hydralazin, Guanethidin, α-Methyldopa, Clonidin oder ein β-Sympathikolytikum, eine antithrombotisch wirksame Verbindung, wie z. B. Acetylsalicylsäure, Sulfinpyrazon, Ticlopidin und Heparinoide, oder ein antihyperurikämisch wirksames Mittel, ein orales Antidiabetikum, ein Geriatrikum oder ein Mittel mit durchblutungssteigernder Wirkung enthalten.

Die folgenden Beispiele erläutern die Herstellung der Verbindungen:

Die NMR-Spektren wurden, falls nicht anders angegeben, in $DCCl_3$ gemessen. Die chemischen Verschiebungen sind in δ-Werten ausgedrückt.

Beispiel 1
exo,endo-2-(1-Imidazolomethyl)-7-isopentyliden-bicyclo[2.2.1]heptan

a) exo,endo-7-Isopentyliden-bicyclo[2.2.1]hept-5-en-2-carbonsäuremethylester

23,3 g (0,175 mol) 6,6-Diethylfulven werden mit 2,4 g (0,280 mol) Acrylsäuremethylester vermischt, mit einer Spatelspitze Hydrochinon versetzt und 30 h auf 60 °C erwärmt. Nach Abziehen des überschüssigen Acrylsäureesters erhält man 30,7 g exo,endo-7-Isopentyliden-bicyclo-[2.2.1]hept-5-en-2-carbonsäure-methylester als Öl.

NMR: 6,0–6,5, m, 2H; 3,6 und 3,7 s, exo und endo $CO_2CH_3$; 1,2–3,5 m, 9H; 0,9, t, 2 $CH_3$

b) exo,endo-7-Isopentyliden-bicyclo[2.2.1]heptan-2-carbonsäuremethylester

30,6 g (0,138 mol) Ester aus a) werden in 100 ml THF gelöst. Nach Zusatz von 2 g Pd/C wird bei Raumtemperatur und Normaldruck bis zur Aufnahme von einem Equivalent Wasserstoff hydriert. Nach Absaugen des Katalysators destilliert man bei 0,2 mm. Man erhält 20,1 g exo,endo-7-Isopentyliden-bicyclo[2.2.1]heptan-2-carbonsäuremethylester vom Siedepunkt 76 °C.

NMR: 3,6 und 3,7, s, exo und endo $CO_2CH_3$; 0,7–3,0, m, 19H.

c) exo,endo-2-Hydroxymethyl-7-isopentyliden-bicyclo[2.2.1]heptan

1,9 (50 mmol) $LiAlH_4$ werden in 100 ml absol. Ether vorgelegt. Dann tropft man unter schwachem Rückfluss 20,0 g (90 mmol) Ester aus b) in 50 ml absol. Ether zu und rührt eine Stunde unter Rückfluss nach. Man hydrolysiert mit verdünnter Salzsäure, trennt die Etherphase ab, wäscht mit Wasser und trocknet über Natriumsulfat. Nach Abziehen des Ethers verbleiben 15,7 g des Alkohols als Öl.

NMR: 3,6 und 3,2 d, exo und endo $CH_2OH$; 1,2–2,8 m, 13H; 0,9, t, 2 $CH_3$

d) exo,endo-7-Isopentyliden-bicyclo[2.2.1]heptan-2-methyl (p-chlorphenyl)sulfonat

Zu 15,6 g (81 mmol) Alkohol aus c) und 18,8 g (89 mmol) p-Chlorbenzolsulfochlorid in 100 ml Methylenchlorid werden bei Raumtemperatur 9,0 g (90 mmol) Triethylamin getropft. Nach 16 h wird mit Wasser versetzt, die organische Phase abgetrennt, einmal mit Wasser gewaschen und getrocknet. Nach dem Einengen am Rotationsverdampfer bleiben 26,6 g exo,endo-7-Isopentyliden-bicyclo[2.2.1]heptan-2-methyl (p-chlorphenyl)sulfonat als Öl zurück.

NMR: 7,4–8,1, m, 4H; 4,0, q und 3,6, d, exo und endo

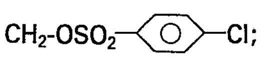; 3,4, m, 19H

e) exo,endo-2-(1-Imidazolomethyl)-7-isopentyliden-bicyclo[2.2.1]heptan

Man legt 6,4 g (72 mmol) Natriumimidazolid in 80 ml DMF vor und tropft bei Raumtemperatur 25,2 g (71 mmol) Sulfonsäureester aus d) in 50 ml DMF zu. Das Reaktionsgemisch wird 16 h bei 60 °C gerührt, abgekühlt und mit Wasser versetzt. Man extrahiert dreimal mit je 150 ml Methylenchlorid, trocknet die organische Phase und engt ein. Nach Reinigung des Rückstandes durch Säulenchromatographie an Kieselgel mit Cyclohexan/Essigsäureethylester (2:1) erhält man 6 g der Titelverbindung als hellgelbes, erstarrendes Öl.

Formel:

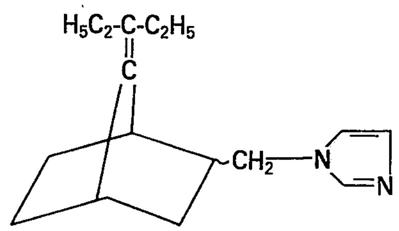

NMR: 7,4, s, 1H; 6,9, d, 2H; 3,9, d und 3,6, 1, exo und endo $CH_2$-Imidazol; 0,5–2,7 m, 19H.

Beispiel 2
endo-2-(1-Imidazolomethyl)-7-cyclohexyliden-bicyclo[2.2.1]heptan

4 g (15,8 mmol) endo-2-(1-Imidazolomethyl)-7-cyclohexyliden-bicyclo[2.2.1]hept-5-en (hergestellt analog Beispiel 1a), c), d), e) aus 6,6-Pentamethylenfulven, Acrylsäuremethylester und Natriumimidazolid; Schmp. 48 °C, vgl. Beispiel 10) werden in 50 ml THF bei Raumtemperatur und Normaldruck in Gegenwart von 0,5 g Pd/C (5%) bis zur Aufnahme von einem Equivalent Wasserstoff hydriert. Der Katalysator wird anschliessend abfiltriert, die Lösung eingeengt und der Rückstand aus Isopropylether umkristallisiert. Man erhält 3,3 g Imidazolderivat vom Schmelzpunkt 95 °C.

Formel:

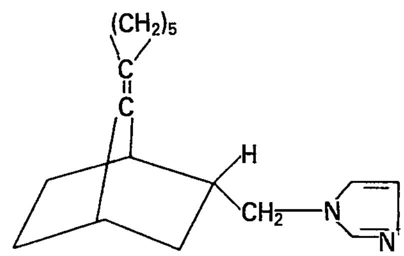

NMR: 7,5, s, 1H; 6,9, d, 2H; 3,9, d und 3,6, q, exo und endo $CH_2$-Imidazol; 0,5–2,7, m, 19H.

Beispiel 3
exo-2-(1-Imidazolomethyl)-7-diphenylmethylenbicyclo[2.2.1]heptan

a) exo und endo 7-Diphenylmethylenbicyclo-[2.2.1]hept-5-en-2-carbonsäure

50 g (0,218 mol) 6,6-Diphenylfulven und 31,3 g (0,435 mol) Acrylsäure werden innig vermischt und 14 Tage bei Raumtemperatur bis zur Entfärbung stehen gelassen. Durch fraktionierendes Umkristallisieren erhält man aus dem weissen festen Rohprodukt mit Ethanol zuerst 35,3 g der

endo-Säure vom Schmelzpunkt 190 °C. Einengen der Mutterlauge und Umkristallisation des Rückstandes aus Cyclohexan ergibt 8,4 g der exo-Säure vom Schmelzpunkt 146 °C.

NMR (endo-Säure): 7,4–7,0, m, 10H; 6,48, q, 1H, 6,28, q, 1H; 3,7–3,9, m, 1H; 3,35–3,55, m, 1H; 3,2, p, H; 2,22 o, 1H; 1,64, q, 1H.

NMR (exo-Säure): 7,0–7,4, m, 10H; 6,42 t, 2H; 3,54–3,64, m, 1H; 3,40–3,52, m, 1H; 2,20–2,55, m, 2H; 1,60, q, 1H.

b) exo-7-Diphenylmethylen-bicyclo[2.2.1]hept-5-en-2-carbonsäuremethylester

7,4 g (24,3 mol) der exo-Säure aus a) werden in 50 ml absolutem Methanol gelöst und mit 0,5 g konzentrierter Schwefelsäure versetzt. Man rührt 2 h bei 40 °C, lässt über Nacht bei Raumtemperatur stehen, versetzt dann mit Wasser und extrahiert mit Methylenchlorid. Nach dem Trocknen und Einengen der organischen Phase verbleiben 7,8 g Methylester als gelbliches Öl.

NMR: 6,9–7,4, m, 10 H; 6,2–6,4, m, 2H; 3,2–3,8, m, 2H; 3,4, s, 3H von $CO_2CH_3$; 2,1–2,6, m, 2H; 1,2–1,8, m, 1H.

c) exo-7-Diphenylmethylen-bicyclo[2.2.1]heptan-2-carbonsäuremethylester

Man löst 7,4 g (23,3 mmol) Ester aus b) in 100 ml THF und hydriert bei Raumtemperatur und Normaldruck in Gegenwart von 5% Pd auf Kohle bis zur Aufnahme von einem Equivalent Wasserstoff. Der Katalysator wird abfiltriert und die Lösung eingeengt. Es verbleiben 8,2 g exo-7-Diphenylmethylen-bicyclo[2.2.1]heptan-2-carbonsäuremethylester als öliger Rückstand.

NMR: 7,0–7,5, m, 10H; 3,4, s, 3H; $CO_2CH_3$; 1,0–2,8, m, 9H.

d) exo-2-Hydroxymethyl-7-diphenylmethylen-bicyclo[2.2.1]heptan

Zu 1,0 g (27,1 mmol) Lithiumaluminiumhydrid in 35 ml trockenem Ether werden 7,9 g (24,8 mmol) Ester aus c) in 50 ml trockenem Ether zugetropft. Man rührt 2 h unter Rückfluss nach, versetzt mit verdünnter Salzsäure und trennt die organische Phase ab, wäscht einmal mit Wasser, trocknet und zieht das Lösungsmittel im Wasserstrahlvakuum ab. Man erhält 7,1 g des Alkohols als Öl.

NMR: 6,7–7,4, m, 10H; 3,3, q, 2H, $CH_2OH$; 0,9–2,5, m, 9H.

e) exo-7-Diphenylmethylen-bicyclo[2.2.1]heptan-2-methyl-(p-chlorphenyl)-sulfonat

7,0 g (24,1 mmol) Alkohol aus d) und 5,7 g (26,6 mmol) p-Chlorbenzolsulfochlorid werden in 50 ml Methylenchlorid vorgelegt. Dazu tropft man bei Raumtemperatur 2,7 g (26,6 mmol) Triethylamin. Nach Stehen über Nacht wird mit Wasser versetzt, die organische Phase wird abgetrennt, einmal mit Wasser gewaschen, getrocknet und eingeengt. Es bleiben 11,8 g Sulfonsäureester als Öl zurück.

NMR: 6,9–7,9, m, 14H; 3,8, q, 2H,

; 0,9–2,5, m, 9H.

f) exo-2-(1-Imidazolomethyl)-7-diphenylmethyl-bicyclo[2.2.1]heptan

Zu 2,3 g (25,8 mmol) Natriumimidazolid in 50 ml DMF werden 10,9 g (23,5 mmol) Sulfonsäureester aus e) in 25 ml DMF gegeben. Man rührt 5 h bei 50 °C, lässt über Nacht stehen, versetzt mit Wasser und extrahiert dreimal mit je 150 ml Methylenchlorid. Die Methylenchloridphase wird noch einmal mit Wasser gewaschen, getrocknet und eingeengt. Der Rückstand (7,2 g) wird mit Cyclohexan/Ethylacetat zur Kristallisation gebracht. Man erhält 3,0 g Imidazolderivat vom Schmelzpunkt 170 °C.

Formel:

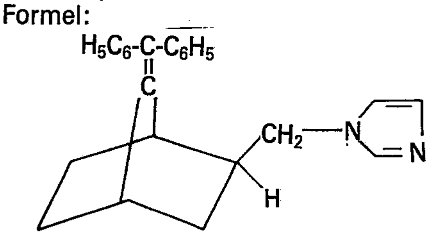

NMR: 7,1–7,5, m, 10H; 6,8, d, 2H; 6,4, s, 1H; 3,9, q, 2H; 1,0–2,6, m, 9H.

Beispiel 4

endo-2-(α-Keto-β-imidazoloethyl)-7-diphenylmethylen-bicyclo[2.2.1]heptan

13,3 g (35 mmol) endo-2-(Bromacetyl)-7-diphenylmethylen-bicyclo[2.2.1]heptan (hergestellt analog Ann. 566, 27 und Synthesis 1976, 194; das erhaltene Rohprodukt wird für die weitere Umsetzung eingesetzt) in 30 ml DMF werden zu 6,0 g (88 mmol) Imidazol in 150 ml DMF getropft und 10 h bei Raumtemperatur gerührt. Man versetzt mit Wasser, extrahiert mit Methylenchlorid, wäscht die organische Phase einmal mit Wasser, trocknet und zieht das Lösungsmittel im Vakuum ab. Aus dem Rückstand werden säulenchromatographisch an Kieselgel 4,2 g Imidazolderivat als hellgelbes Öl erhalten.

Formel:

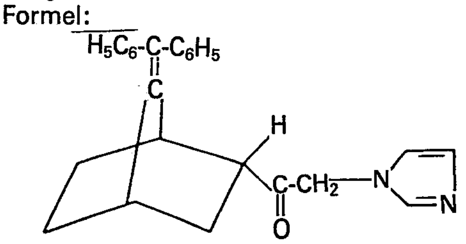

NMR: 6,0–7,4, m, 1H; 6,6, d, 2H, 4,5, s, 2H; 1,0–3,0, m, 9H.

Beispiel 5

exo,endo-2-(1-Imidazolyl)-7-diphenylmethylenbicyclo[2.2.1]heptan

a) exo,endo-2-Chlor-2-cyano-7-diphenylmethylenbicyclo[2.2.1]hept-5-en

46 g (0,2 mol) 6,6-Diphenylfulven und 17,6 g (0,2 mol) α-Chloracrylnitril werden in 120 ml Toluol gelöst und 20 h bei 80 °C gerührt. Man zieht das Lösungsmittel im Wasserstrahlvakuum ab und kristallisiert aus Isopropanol um. Man erhält

37,2 g exo,endo-2-Chlor-2-cyano-7-diphenylmethylenbicyclo[2.2.1]hept-5-en als farblose Kristalle vom Schmelzpunkt 135 °C.

NMR: 7,0–7,4, m, 10H; 6,3–6,8, m, 2H; 3,8–4,0, m, 1H; 3,4–3,7, m, 1H; 2,9, q, 1H, 1,8, d, 1H.

b) exo,endo-2-Chlor-2-cyano-7-diphenylmethylenbicyclo[2.2.1]heptan

17,5 g (55 mmol) Bicycloheptenderivat aus a) werden in Gegenwart von 1 g Pd/C bei Raumtemperatur und Normaldruck bis zur Aufnahme von 1235 ml Wasserstoff hydriert. Nach Abfiltrieren des Katalysators und Entfernen des Lösungsmittels verbleiben 17,6 g Bicycloheptanderivat als Feststoff vom Schmelzpunkt 84–87 °C.

NMR: 7,0–7,4, m, 10H; 1,3–3,2, m, 8H.

c) 7-Diphenylmethylen-bicyclo[2.2.1]heptan-2-on

17,5 g (55 mmol) des Bicycloheptanderivats aus b) werden in 170 ml DMSO gelöst. Dazu werden 7,8 g (139,3 mmol) KOH in 30 ml Wasser gegeben. Dann wird drei Stunden bei Raumtemperatur gerührt. Nach Versetzen mit 800 ml Wasser wird mit Methylenchlorid extrahiert, die organische Phase wird getrocknet und eingeengt. Der Rückstand wird aus Isopropylether umkristallisiert. Man erhält 13,7 g Keton vom Schmelzpunkt 88 °C.

NMR: 7,0–7,4, m, 10H; 3,1–3,4, m, 2H; 1,5–2,5, m, 6H.

d) exo,endo-7-Diphenylmethylen-bicyclo[2.2.1]-heptan-2-ol

12,0 g (44 mmol) des Ketons aus c) werden in einem Gemisch aus 50 ml Ethanol und 30 ml Tetrahydrofuran gelöst und mit 1,2 g (33 mmol) $NaBH_4$ fünf Stunden bei Raumtemperatur reduziert. Nach Versetzen mit Wasser wird mit Methylenchlorid extrahiert. Die organische Phase wird getrocknet und eingeengt. Man erhält 11,8 g des Alkohols als Öl.

NMR: 7,0–7,4, m, 10H; 3,5–4,5, m, exo- und endo-2-H; 1,0–2,8, m, 8H.

e) exo,endo-7-Diphenylmethylen-bicyclo[2.2.1]-heptan-2-methylsulfonat

11,5 g (42,8 mmol) des Alkohols aus d) und 5,4 g (47,0 mmol) Methansulfonsäurechlorid werden in 100 ml Methylenchlorid langsam mit 4,8 g (48,0 mmol) Triäthylamin versetzt und 16 h bei Raumtemperatur gerührt. Nach Zugabe von 500 ml Wasser wird die organische Phase abgetrennt, getrocknet und eingeengt. Man erhält 14,7 g Sulfonsäureester als Öl.

NMR: 7,0–7,4, m, 10H; 4,8–5,2, m, exo- und endo-2-H; 2,9, s, $CH_3$; 1,0–3,1, m, 8H.

f) exo,endo-2-(1-Imidazolyl)-7-diphenylmethylenbicyclo[2.2.1]heptan

Aus 3,1 g (45,7 mmol) Imidazol und 1,4 g (45,7 mmol) 80%igem Natriumhydrid wird in 50 ml DMF eine Na-Imidazolid-Lösung hergestellt. Zu dieser tropft man 14,7 g (41,5 mmol) des Sulfonsäureesters aus e) in 50 ml DMF zu und rührt 7 Tage bei 100 °C. Nach Versetzen mit Wasser wird mit Methylenchlorid extrahiert. Die organische Phase wird getrocknet und eingeengt. Der ölige Rückstand (12,7 g) wird an Kieselgel chromatographiert (Laufmittel: Cyclohexan/Essigsäureethylester wie 3:1). Man eluiert 1,2 g des gewünschten Imidazolderivats als Öl, aus dem mit Chlorwasserstoff in Ether 1,0 g des entsprechenden Salzes vom Schmelzpunkt 160 °C erhalten wird.

Formel:

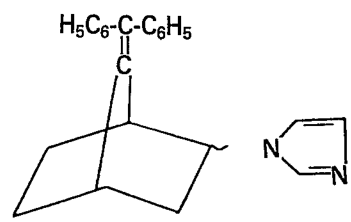

NMR: 8,8–9,0, s, 1H; 6,5–7,5, m, 13H; 5,4–5,7, m, 1H; 1,0–2,8, m, 8H.

In analoger Weise werden folgende Verbindungen der Formel I hergestellt (Beispiele 6 bis 23)

| Nr | $R^1$ | $R^2$ | A | B | Konfig. | n | phys. Daten |
|---|---|---|---|---|---|---|---|
| 6 | $CH_3$ | $CH_3$ | = | – | exo/endo | 1 | 158 °C (+HCl) |
| 7 | $CH_3$ | $CH_3$ | – | – | exo/endo | 1 | Öl, NMR: 7,4, s, 1H; 6,9, d, 2H; 3,9 u, 3,6 d, exo u. endo-$CH_2$-Imidazol; 0,7–2,8, m, 9H; 2,7, s $CH_3$; 2,6, d, $CH_3$ |
| 8 | $(CH_2)_4$ | | = | – | exo/endo | 1 | Öl, NMR: 7,4, s, 1H; 6,9, d, 2H; 6,0–6,5, m, 2H; 3,5–4,0, m, exo u. endo-$CH_2$-Imidazol; 0,5–3,1, m, 13H |
| 9 | $(CH_2)_4$ | | – | – | exo/endo | 1 | Öl, NMR: 7,4, s, 1H; 6,9, d, 2H; 3,4–4,0, m, exo u. endo-$CH_2$-Imidazol; 0,5–2,5, m, 17H |
| 10 | $(CH_2)_5$ | | = | – | endo | 1 | 48 °C |
| 11 | $C_6H_5$<br>$(CH_2)_2$-CH-$(CH_2)_2$ | | = | – | endo | 1 | 161 °C |
| 12 | $C_6H_5$<br>$(CH_2)_2$-CH$(CH_2)_2$ | | – | – | endo | 1 | 130 °C |
| 13 | 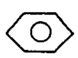 | 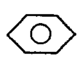 | – | – | endo | 0 | 160 °C (+HCl) |
| 14 | 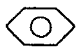 | 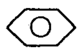 | = | – | endo | 1 | 140 °C |
| 15 | 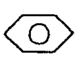 | 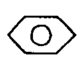 | – | – | endo | 1 | 146 °C |
| 16 | 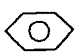 | 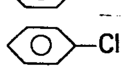 | – | – | endo | 1 | 165 °C (+HCl) |
| 17 | 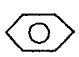 | 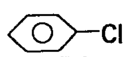 | – | – | exo | 1 | 162 °C (+HCl) |
| 18* | 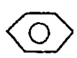 | 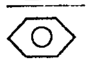 | – | CHOH | endo | 1 | 160 °C (+HCl) |
| 19 | 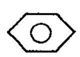 | 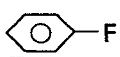 | – | – | exo | 1 | 210 °C (+HCl) |
| 20 | 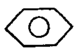 | 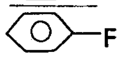 | – | – | endo | 1 | 144 °C |
| 21 | 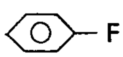 | 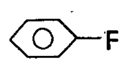 | – | – | endo | 1 | 152 °C |
| 22 | 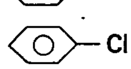 | 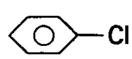 | – | – | endo | 1 | 198 °C (+HCl) |
| 23 | 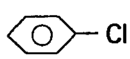 | 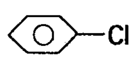 | – | – | exo | 1 | amorph, NMR: 6,8–7,5, 10H; 6,4, s, 1H; 3,5, q, 2H, exo-$CH_2$-Imidazol; 0,7–2,8, m, 9H. |

Beispiel 24
exo-2-Carbonylimidazolyl-7-diphenylmethylenbicyclo[2.2.1]heptan

Bei den Verbindungen 16, 17, 19 und 20 kann auch $R^2$ unsubstituiertes und $R^1$ mit Chlor bzw. Fluor substituiertes Phenyl sein.

*) wurde durch Reduktion der Verbindung gemäss Beispiel 4 mit $NaBH_4$ in an sich bekannter Weise erhalten.

a) exo-7-Diphenylmethylenbicyclo[2.2.1]heptan-2-carbonsäure

8 g (26,5 mmol) exo-7-Diphenylmethylenbicyclo[2.2.1]hept-5-en-2-carbonsäure wurden in 80 ml Tetrahydrofuran gelöst und bei Normaldruck und Raumtemperatur in Gegenwart von 1 g Pd/C bis zur Aufnahme von einem Äquivalent Wasserstoff hydriert. Nach Abfiltrieren des Katalysators und Einengen der Lösung verblieben 8,1 g der hydrierten Säure als Öl.

NMR: 11,2, 1H; 6,9–7,4, m, 10H; 3,5–3,8, m, 2H; 1,0–3,2, m, 7H.

b) exo-2-Carbonylimidazolyl-7-diphenylmethylenbicyclo[2.2.1]heptan

8,1 g (26,5 mmol) der Säure aus a) wurden in 60 ml Tetrahydrofuran vorgelegt. Es wurden 2 Tropfen Pyridin zugegeben und 5,5 g (46 mmol) Thionylchlorid zugetropft. Man erhitzte zwei Stunden zum Rückfluss, zog das Lösungsmittel im Vakuum ab und erhielt 9,4 g rohes Säurechlorid. Dieses löste man wieder in 50 ml Tetrahydrofuran und tropfte bei 0–5 °C 6,9 g (102 mmol) Imidazol in Tetrahydrofuran zu, rührte 24 h bei Raumtemperatur und arbeitete mit Wasser und Methylenchlorid auf. Man erhielt 8,8 g Öl, das im Kugelrohr (250 °C, 0,3 mbar) destilliert wurde. Es ergaben sich so 4,5 g reines Imidazolderivat.

Formel:

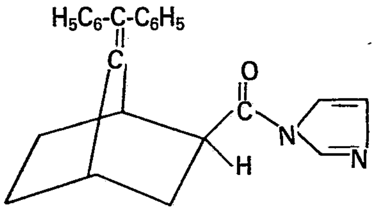

NMR: 8,1, s, 1H; 6,8–7,5, m, 10H; 3,2–3,8, m, 1H; 1,0–3,0, m, 8H.

Beispiel 25

Exo,endo-2-(1-Imidazoloethyl)-7-diphenylmethylenbicyclo[2.2.1]heptan

a) E,Z-7-Diphenylmethylenbicyclo[2.2.1]heptan-2-methylencarbonsäureethylester

3,0 g (0,1 mol) 80%iges Natriumhydrid wurden in 250 ml Dimethoxyethan vorgelegt und 22,5 g (0,1 mol) Triethylphosphonacetat in Dimethoxyethan zugetropft. Nach einer Stunde tropfte man 27,5 g (0,1 mol) Keton, erhalten gemäss Beispiel 5c) in Dimethoxyethan zu, rührte 3 h bei 50 °C nach und arbeitete mit Wasser/Methylenchlorid auf. Man erhielt 35 g Produkt als Öl.

NMR: 7,0–7,4, m, 10H; 5,6 und 5,8, m, 1H; 4,2, q, 2H; 3,3, m, 1H; 1,0–3,0, m, 7H; 1,3, t, 3H.

b) E,Z-7-Diphenylmethylenbicyclo[2.2.1]heptan-2-(ethyliden-2-ol)

Zu 3,8 g (0,1 mol) Lithiumaluminiumhydrid in 100 ml THF tropfte man 23 g (0,067 mol) Ester aus a) in THF, rührte 8 h bei 70 °C und arbeitete mit verdünnter Salzsäure/Methylenchlorid auf. Nach Entfernen des Lösungsmittels blieben 21,6 g Rohprodukt, aus dem mittels Säulenchromatographie (Kieselgel, Cyclohexan/Ethylenacetat = 5:1) 12,5 g Alkohol isoliert wurden.

NMR: 7,0–7,4, m, 10H; 5,4–5,6, m, 1H; 4,1, d, 2H; 3,2, m, 1H; 2,9, m, 1H; 1,1–2,6, m, 6H.

c) Exo-endo-7-Diphenylmethylenbicyclo[2.2.1]-heptan-2-(ethyl-2-ol)

6,8 g (22,5 mmol) Alkohol aus b) wurden in 100 ml THF gelöst und in Gegenwart von 1 g Pd/C bei Raumtemperatur und Normaldruck bis zur Aufnahme von einem Equivalent Wasserstoff hydriert. Nach Abfiltrieren des Katalysators und Entfernen des Lösungsmittels blieben 6,7 g Öl zurück.

NMR: 6,9–7,4, m, 10H; 3,3–3,8, m, 1H; 0,5–3,1, m, 12H.

Der Alkohol aus 25c) wurde analog Beispiel 3e), f) zum Imidazolderivat weiter umgesetzt. Man erhielt 0,8 g eines farblosen Öls.

Formel:

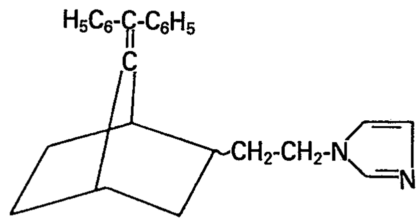

NMR: 6,6–7,5, m, 13H; 3,6–4,0, m, 2H; 0,6–2,8, m, 11H.

**Patentansprüche für die Vertragsstaaten BE, CH, DE, FR, GB, IT, LI, LU, NL, SE**

1. N-Bicycloheptyl und N-Bicycloheptenyl-imidazole der Formel I

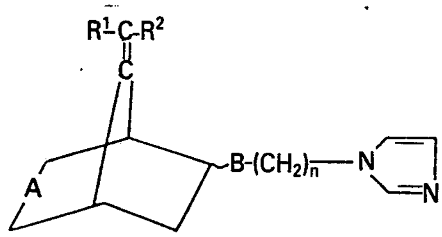

I

worin

$R^1$ und $R^2$ gleich oder verschieden sind und Alkyl mit 1 bis 10 C-Atomen, Cycloalkyl mit 4 bis 10 C-Atomen, Phenyl, das gegebenenfalls mono- oder disubstituiert ist mit Halogen, $(C_1–C_4)$-Alkyl, $(C_1–C_4)$-Alkoxy, Trifluormethyl, Hydroxy, Amino, $(C_1–C_4)$-Alkylamino oder $(C_1–C_4)$-Dialkylamino, wobei im Falle der Disubstitution die Substituenten gleich oder verschieden sind, Naphthyl oder Phenylalkyl mit 1 bis 4 Alkylkohlenstoffatomen bedeuten, oder $R^1$ und $R^2$ gemeinsam für eine gegebenenfalls mit Phenyl substituierte $(CH_2)_m$-Brücke, wobei m eine Zahl von 3 bis 10 bedeutet, stehen

A eine Einfach- oder Doppelbindung darstellt,

B eine einfache Bindung, oder, falls n für 0 oder 1 steht, auch die $-\underset{\underset{O}{\|}}{C}-$-Gruppe oder falls n für 1 steht, die -CHOH-Gruppe bedeutet und

n für 0, 1, 2, 3 oder 4 steht,

sowie deren physiologisch verträglichen Säureadditionssalze.

2. Verbindungen gemäss Anspruch 1, dadurch gekennzeichnet, dass in Formel I $R^1$ und $R^2$ gleich oder verschieden sind und Phenyl, 4-Chlorphenyl oder 4-Fluorphenyl, A eine Einfachbindung bedeutet, n für 0 oder 1 steht und B eine Einfachbindung oder die CO-Gruppe bedeutet.

3. Verbindungen gemäss Anspruch 2, dadurch gekennzeichnet, dass in Formel = B die CO-Gruppe bedeutet.

4. Verfahren zur Herstellung von Verbindungen gemäss Anspruch 1, dadurch gekennzeichnet, dass man

a) einen Alkohol der Formel II

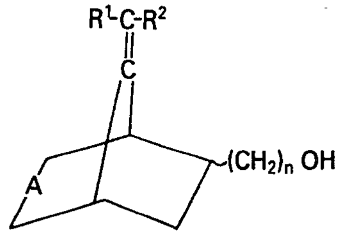
II

worin A, $R^1$, $R^2$ und n die zu Formel I angegebenen Bedeutungen haben, nach an sich bekannten Methoden in eine Verbindung der Formel III

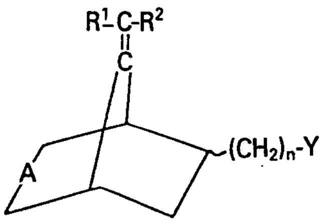
III

worin A, $R^1$, $R^2$ und n der zu Formel I angegebenen Bedeutungen haben und Y eine leaving group bedeutet, überführt und mit Imidazol oder einem Imidazolsalz der Formel IV

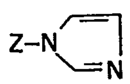
IV

worin Z Wasserstoff oder Alkalimetall bedeutet zu einer Verbindung der Formel I, worin $R^1$, $R^2$, A und n die angegebenen Bedeutungen haben und B eine einfache Bindung darstellt, umsetzt, und gegebenenfalls in einer erhaltenen Verbindung, worin A eine Doppelbindung darstellt, die Doppelbindung hydriert, wobei die Hydrierung entweder an einem Zwischenprodukt oder an einer Verbindung der Formel I (A = Doppelbindung) durchgeführt wird, oder

b) ein Halogenid der Formel V

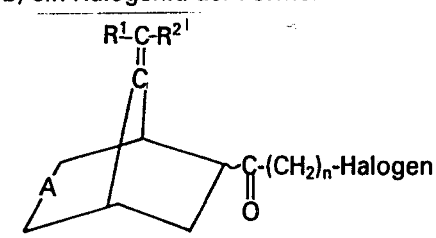
V

worin A, $R^1$ und $R^2$ die zu Formel I angegebenen Bedeutungen haben, n für 0 oder 1 steht und Halogen für Brom oder Chlor steht, mit Imidazol oder einem Imidazolsalz der Formel IV zu einer Verbindung der Formel I, worin A, $R^1$ und $R^2$ die zu Formel I angegebenen Bedeutungen haben, B die $\underset{\underset{O}{\|}}{C}$-Gruppe, und n = 0 oder 1 sind, umsetzt und gegebenenfalls zu einer Verbindung, worin B die CHOH-Gruppe bedeutet, reduziert und gewünschtenfalls die nach a) oder b) erhaltenen Verbindungen mit einer Säure in die physiologisch verträglichen Säureadditionssalze überführt.

5. Pharmazeutisches Präparat, enthaltend eine Verbindung gemäss Anspruch 1.

6. Verfahren zur Herstellung eines pharmazeutischen Präparates, enthaltend eine Verbindung gemäss Anspruch 1, dadurch gekennzeichnet, dass man sie durch Zusatz von Hilfsstoffen in eine geeignete Darreichungsform überführt.

7. Verbindungen gemäss Anspruch 1 zur Behandlung von Störungen des Serumlipoproteinspektrums.

**Patentansprüche für den Vertragsstaat AT**

1. Verfahren zur Herstellung von N-Bicycloheptyl und N-Bicycloheptenyl-imidazole der Formel I

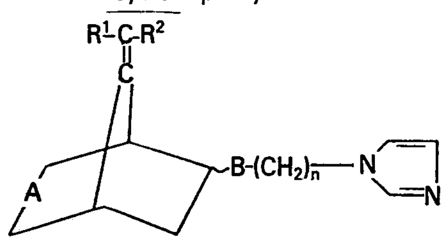
I

worin

$R^1$ und $R^2$ gleich oder verschieden sind und Alkyl mit 1 bis 10 C-Atomen, Cycloalkyl mit 4 bis 10 C-Atomen, Phenyl, das gegebenenfalls mono- oder disubstituiert ist mit Halogen, $(C_1–C_4)$-Alkyl, $(C_1–C_4)$-Alkoxy, Trifluormethyl, Hydroxy, Amino, $(C_1–C_4)$-Alkylamino oder $(C_1–C_4)$-Dialkylamino, wobei im Falle der Disubstitution die Substituenten gleich oder verschieden sind, Naphthyl oder Phenylalkyl mit 1 bis 4 Alkylkohlenstoffatomen bedeuten, oder $R^1$ und $R^2$ gemeinsam für eine gegebenenfalls mit Phenyl substituierte $(CH_2)_m$-Brücke, wobei m eine Zahl von 3 bis 10 bedeutet, stehen

A eine Einfach- oder Doppelbindung darstellt,

B eine einfache Bindung, oder, falls n für 0 oder 1 steht, auch die -$\underset{\underset{O}{\|}}{C}$-Gruppe oder falls n für 1 steht, die -CHOH-Gruppe bedeutet und

n für 0, 1, 2, 3 oder 4 steht,

sowie deren physiologisch verträglichen Säureadditionssalze, dadurch gekennzeichnet, dass man

a) einen Alkohol der Formel II

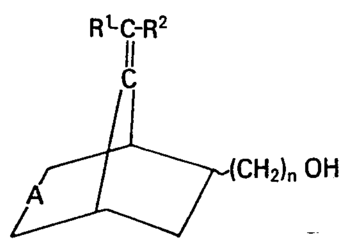
II

worin A, $R^1$, $R^2$ und n die zu Formel I angegebenen

Bedeutungen haben, nach an sich bekannten Methoden in eine Verbindung der Formel III

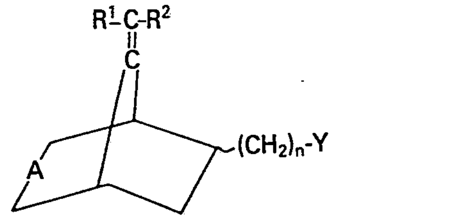

III

worin A, $R^1$, $R^2$ und n der zu Formel I angegebenen Bedeutungen haben und Y eine leaving group bedeutet, überführt und mit Imidazol oder einem Imidazolsalz der Formel IV

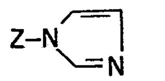

IV

worin Z Wasserstoff oder Alkalimetall bedeutet zu einer Verbindung der Formel I, worin $R^1$, $R^2$, A und n die angegebenen Bedeutungen haben und B eine einfache Bindung darstellt, umsetzt, und gegebenenfalls in einer erhaltenen Verbindung, worin A eine Doppelbindung darstellt, die Doppelbindung hydriert, wobei die Hydrierung entweder an einem Zwischenprodukt oder an einer Verbindung der Formel I (A = Doppelbindung) durchgeführt wird, oder

b) ein Halogenid der Formel V

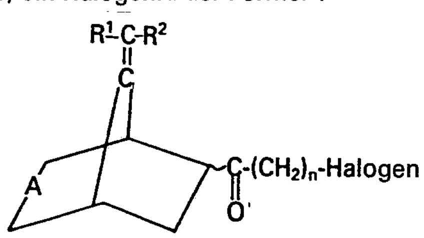

V

worin A, $R^1$ und $R^2$ die zu Formel I angegebenen Bedeutungen haben, n für 0 oder 1 steht und Halogen für Brom oder Chlor steht, mit Imidazol oder einem Imidazolsalz der Formel IV zu einer Verbindung der Formel I, worin A, $R^1$ und $R^2$ die zu Formel I angegebenen Bedeutungen haben, B die $\underset{\underset{O}{\|}}{C}$-Gruppe, und n = 0 oder 1 sind, umsetzt und gegebenenfalls zu einer Verbindung, worin B die CHOH-Gruppe bedeutet, reduziert und gewünschtenfalls die nach a) oder b) erhaltenen Verbindungen mit einer Säure in die physiologisch verträglichen Säureadditionssalze überführt.

2. Verfahren gemäss Anspruch 1, dadurch gekennzeichnet, dass man eine Verbindung der Formel I, worin $R^1$ und $R^2$ gleich oder verschieden sind und Phenyl, 4-Chlorphenyl oder 4-Fluorphenyl, A eine Einfachbindung bedeutet, n für 0 oder 1 steht und B eine Einfachbindung oder die CO-Gruppe bedeutet.

3. Verfahren gemäss Anspruch 2, dadurch gekennzeichnet, dass B die CO-Gruppe bedeutet.

4. Verfahren zur Herstellung eines pharmazeutischen Präparates, enthaltend eine Verbindung der Formel I gemäss Anspruch 1 oder ein physiologisch verträgliches Salz einer solchen Verbindung, dadurch gekennzeichnet, dass man sie durch Zusatz von Hilfsstoffen in eine geeignete Darreichungsform überführt.

**Claims for the contracting States: BE, CH, DE, FR, GB, IT, LI, LU, NL, SE**

1. N-Bicycloheptyl and N-bicycloheptenyl-imidazoles of the formula I

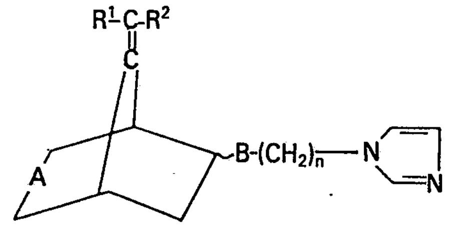

I

in which $R^1$ and $R^2$ are identical or different and denote alkyl having 1 to 10 carbon atoms, cycloalkyl having 4 to 10 carbon atoms, phenyl which is optionally mono- or disubstituted by halogen, $(C_1-C_4)$-alkyl, $(C_1-C_4)$-alkoxy, trifluoromethyl, hydroxyl, amino, $(C_1-C_4)$-alkylamino or $(C_1-C_4)$-dialkylamino, the substituents in the case of disubstitution being identical or different, naphthyl or phenylalkyl having 1 to 4 alkyl carbon atoms, or $R^1$ and $R^2$ together represent a $(CH_2)_m$ bridge which is optionally substituted by phenyl, m denoting a number from 3 to 10,

A represents a single or double bond,

B denotes a single bond or, if n represents 0 or 1, also $-\underset{\underset{O}{\|}}{C}$-group or, if n represents 1, the -CHOH-group, and

n represents 0, 1, 2, 3 or 4,

and their physiologically tolerated acid addition salts.

2. Compounds as claimed in claim 1, wherein $R^1$ and $R^2$ in formula I are identical or different and denote phenyl, 4-chlorophenyl or 4-fluorophenyl, A denotes a single bond, n represents 0 or 1, and B denotes a single bond or the CO-group.

3. Compounds as claimed in claim 2, wherein B in formula I denotes the CO-group.

4. A process for the preparation of compounds as claimed in claim 1, which comprises

a) converting an alcohol of the formula II

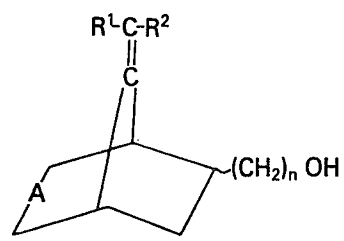

II

in which A, $R^1$, $R^2$ and n have the meanings indicated for formula I, by methods known per se into

a compound of the formula III

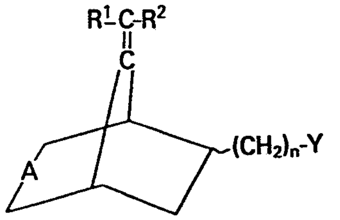

III

in which A, $R^1$, $R^2$ and n have the meanings indicated for formula I, and Y denotes a leaving group, and reacting the latter with imidazole or an imidazole salt of the formula IV

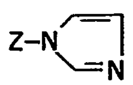

IV

in which Z denotes hydrogen or alkali metal, to give a compound of the formula I in which $R^1$, $R^2$, A and n have the meanings indicated, and B represents a single bond, and, where appropriate, hydrogenating the double bond in a resulting compound in which A represents a double bond, the hydrogenation being carried out either on an intermediate or on a compound of the formula I (A = double bond), or

b) reacting a halide of the formula V

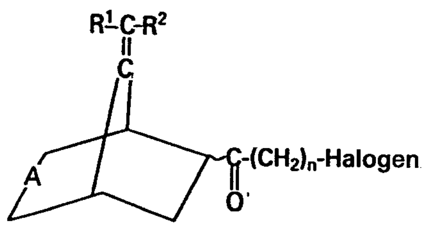

V

in which A, $R^1$ and $R^2$ have the meanings indicated for formula I, n represents 0 or 1, and halogen represents bromine or chlorine, with imidazole or an imidazole salt of the formula IV, to give a compound of the formula I in which A, $R^1$ and $R^2$ have the meanings indicated for formula I, B is the $-\underset{\underset{O}{\|}}{C}-$group, and n is 0 or 1, and, where appropriate, reducing the latter to give a compound in which B denotes the CHOH group and, if desired, converting with an acid the compounds obtained by a) or b) into the physiologically tolerated acid addition salts.

5. A pharmaceutical product containing a compound as claimed in claim 1.

6. A process for the preparation of a pharmaceutical product containing a compound as claimed in claim 1, which comprises converting it into a suitable administration form by addition of auxiliaries.

7. Compounds as claimed in claim 1 for the treatment of disturbances of the serum lipoprotein spectrum.

**Claims for the contracting State: AT**

1. A process for the preparation of N-bicycloheptyl and n-bicycloheptenyl-imidazoles of the formula I

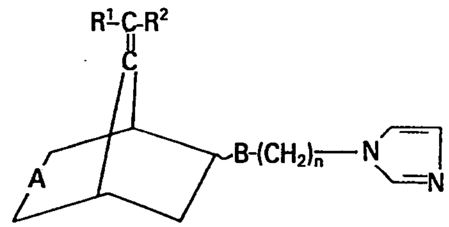

I

in which $R^1$ and $R^2$ are identical or different and denote alkyl having 1 to 10 carbon atoms, cycloalkyl having 4 to 10 carbon atoms, phenyl which is optionally mono- or disubstituted by halogen, $(C_1-C_4)$-alkyl, $(C_1-C_4)$-alkoxy, trifluoromethyl, hydroxyl, amino, $(C_1-C_4)$-alkylamino or $(C_1-C_4)$-dialkylamino, the substituents in the case of disubstitution being identical or different, naphthyl or phenylalkyl having 1 to 4 alkyl carbon atoms, or $R^1$ and $R^2$ together represent a $(CH_2)_m$ bridge which is optionally substituted by phenyl, m denoting a number from 3 to 10,

A represents a single or double bond,

B denotes a single bond or, if n represents 0 or 1, also $-\underset{\underset{O}{\|}}{C}-$group or, if n represents 1, the -CHOH-group, and

n represents 0, 1, 2, 3 or 4,

and their physiologically tolerated acid addition salts which comprises

a) converting an alcohol of the formula II

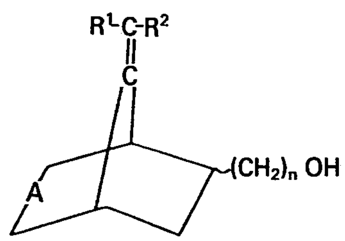

II

in which A, $R^1$, $R^2$ and n have the meanings indicated for formula I, by methods known per se into a compound of the formula III

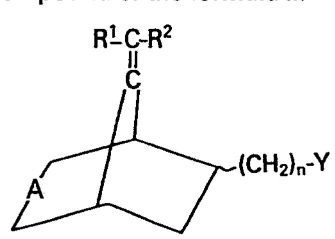

III

in which A, $R^1$, $R^2$ and n have the meanings indicated for formula I, and Y denotes a leaving group, and reacting the latter with imidazole or an imidazole salt of the formula IV

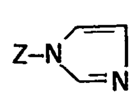

IV

in which Z denotes hydrogen or alkali metal, to give a compound of the formula I in which $R^1$, $R^2$, A and n have the meanings indicated, and B represents a single bond, and, where appropriate, hydrogenating the double bond in a resulting compound in which A represents a double bond, the hydrogenation being carried out either on a intermediate or on a compound of the formula I (A = double bond), or

b) reacting a halide of the formula V

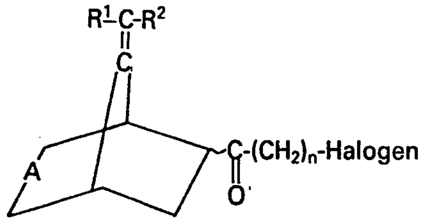

V

in which A, $R^1$ and $R^2$ have the meanings indicated for formula I, n represents 0 or 1, and halogen represents bromine or chlorine, with imidazole or an imidazole salt of the formula IV, to give a compound of the formula I in which A, $R^1$ and $R^2$ have the meanings indicated for formula I, B is the $-\underset{\underset{O}{\|}}{C}-$group, and n is 0 or 1, and, where appropriate, reducing the latter to give a compound in which B denotes the CHOH group and, if desired, converting with an acid the compounds obtained by a) or b) into the physiologically tolerated acid addition salts.

2. A process as claimed in claim 1, wherein $R^1$ and $R^2$ in formula I are identical or different and denote phenyl, 4-chlorophenyl or 4-fluorophenyl, A denotes a single bond, n represents 0 or 1, and B denotes a single bond or the CO group.

3. A process as claimed in claim 2, wherein B denotes the CO group.

4. A process for the preparation of a pharmaceutical product containing a compound of formula I as claimed in claim 1 or a physiologically tolerated salt of such a compound, which comprises converting it into a suitable administration form by addition of auxiliaries.

**Revendications pour les Etats contractants: BE, CH, DE, FR, GB, IT, LI, LU, NL, SE**

1. N-bicycloheptyl- et N-bicycloheptényl-imidazoles de formule I

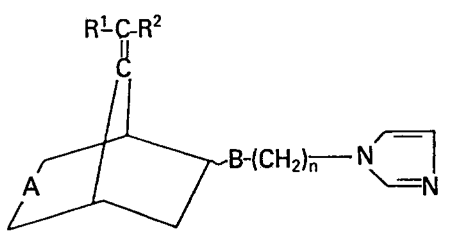

I

dans laquelle

$R^1$ et $R^2$ sont identiques ou différents et représentent un groupe alkyle à 1 à 10 atomes de C, cycloalkyle à 4 à 10 atomes de C, phényle, qui est éventuellement mono- ou disubstitué par un halogène, un groupe alkyle en ($C_1$–$C_4$), alcoxy en ($C_1$–$C_4$), trifluorométhyle, hydroxy, amino, alkylamino en ($C_1$–$C_4$) ou dialkylamino en ($C_1$–$C_4$), les substituants étant identiques ou différents dans le cas de la disubstitution, naphthyle ou phénylalkyle à 1 à 4 atomes de carbone dans la partie alkyle, ou bien $R^2$ et $R^1$ représentent ensemble un pont $(CH_2)_m$ éventuellement substitué par un groupe phényle, m désignant un nombre de 3 à 10,

A représente une liaison simple ou une double liaison,

B représente une liaison simple ou, au cas où n représente 0 ou 1, le groupe $-\underset{\underset{O}{\|}}{C}-$ ou, au cas où n représente 1, le groupe -CHOH- et

n représente 0, 1, 2, 3 ou 4,

ainsi que leurs sels d'addition à un acide physiologiquement acceptables.

2. Composés selon la revendication 1, caractérisés en ce que dans la formule I, $R^1$ et $R^2$ sont identiques ou différents et représentent un groupe phényle, 4-chlorophényle ou 4-fluorophényle, A représente une liaison simple, n représente 0 ou 1 et B représente une liaison simple ou le groupe CO.

3. Composés selon la revendication 2, caractérisés en ce que dans la formule I, B représente le groupe CO.

4. Procédé de préparation de composés selon la revendication 1, caractérisé en ce que

a) on convertit un alcool de formule II

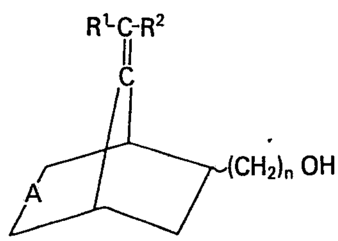

II

dans laquelle A, $R^1$, $R^2$ et n ont les significations indiquées pour la formule I, par des méthodes connues en soi, en un composé de formule III

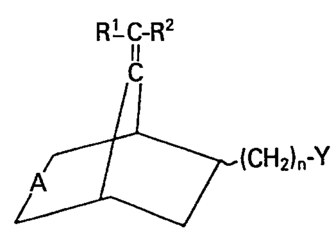

III

dans laquelle A, $R^1$, $R^2$ et n ont les significations indiquées pour la formule I et Y représente un leaving group et on le fait réagir avec l'imidazole ou avec un sel d'imidazole de formule IV

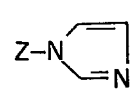

IV

dans laquelle Z représente l'hydrogène ou un métal alcalin, pour obtenir un composé de formule I dans laquelle $R^1$, $R^2$, A et n ont les significations indiquées et B représente une liaison simple et éventuellement, dans un composé obtenu, dans lequel A représente une double liaison, on hydrogène la double liaison, l'hydrogénation étant réalisée soit sur un produit intermédiaire soit sur un composé de formule I (A = double liaison), ou

b) on fait réagir un halogénure de formule V

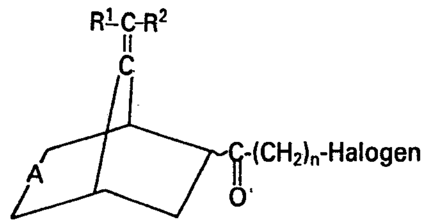

V

dans laquelle A, $R^1$ et $R^2$ ont les significations indiquées pour la formule I, n représente 0 ou 1 et halogène représente le brome ou le chlore, avec l'imidazole ou un sel d'imidazole de formule IV pour obtenir un composé de formule I, dans laquelle A, $R^1$ et $R^2$ ont les significations indiquées pour la formule I, B est le groupe $\underset{\underset{O}{\|}}{C}$ et n est égal à 0 ou 1, et éventuellement on le réduit en un composé dans lequel B représente le groupe CHOH et si on le désire, on convertit les composés obtenus suivants a) ou b) avec un acide en les sels d'addition d'acides physiologiquement acceptables.

5. Préparation pharmaceutique contenant un composé selon la revendication 1.

6. Procédé de préparation d'une préparation pharmaceutique contenant un composé selon la revendication 1, caractérisé en ce qu'on l'amène à une forme d'administration appropriée par addition de substances auxiliaires.

7. Composés selon la revendication 1, destinés au traitement des troubles du spectre des lipoprotéines du sérum.

**Revendications pour l'Etat contractant AT**

1. Procédé de préparation de N-bicycloheptyl- et N-bicycloheptényl-imidazoles de formule I

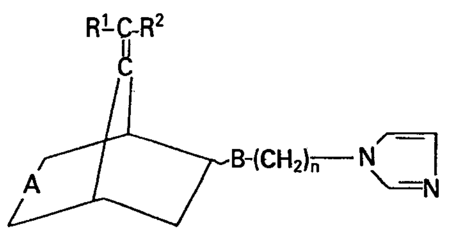

I

dans laquelle

$R^1$ et $R^2$ sont identiques ou différents et représentent un groupe alkyle à 1 à 10 atomes de C, cycloalkyle à 4 à 10 atomes de C, phényle, qui est éventuellement mono- ou disubstitué par un halogène, un groupe alkyle en ($C_1$–$C_4$), alcoxy en ($C_1$–$C_4$), trifluorométhyle, hydroxy, amino, alkylamino en ($C_1$–$C_4$) ou dialkylamino en ($C_1$–$C_4$), les substituants étant identiques ou différents dans le cas de la disubstitution, naphthyle ou phénylalkyle à 1 à 4 atomes de carbone dans la partie alkyle, ou bien $R^1$ et $R^2$ représentent ensemble un pont $(CH_2)_m$ éventuellement substitué par un groupe phényle, m désignant un nombre de 3 à 10,

A représente une liaison simple ou une double liaison,

B représente une liaison simple ou, au cas où n représente 0 ou 1, le groupe $\underset{\underset{O}{\|}}{-C-}$ ou, au cas où n représente 1, le groupe -CHOH- et

n représente 0, 1, 2, 3 ou 4,

ainsi que leurs sels d'addition à un acide physiologiquement acceptables, caractérisé en ce que

a) on convertit un alcool de formule II

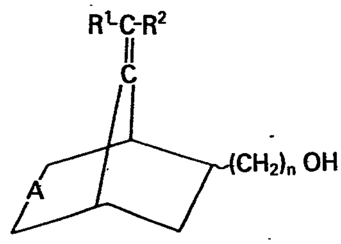

II

dans laquelle A, $R^1$, $R^2$ et n ont les significations indiquées pour la formule I, par des méthodes connues en soi, en un composé de formule III

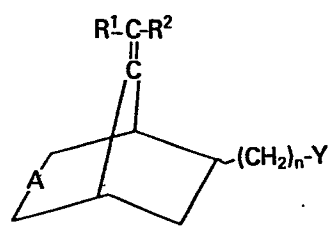

III

dans laquelle A, $R^1$, $R^2$ et n ont les significations indiquées pour la formule I et Y représente un leaving group et on le fait réagir avec l'imidazole ou avec un sel d'imidazole de formule IV

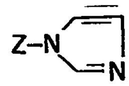

IV

dans laquelle Z représente l'hydrogène ou un métal alcalin, pour obtenir un composé de formule I dans laquelle $R^1$, $R^2$, A et n ont les significations indiquées et B représente une liaison simple et éventuellement, dans un composé obtenu, dans lequel A représente une double liaison, on hydrogène la double liaison, l'hydrogénation étant réalisée soit sur un produit intermédiaire soit sur un composé de formule I (A = double liaison), ou

b) on fait réagir un halogénure de formule V

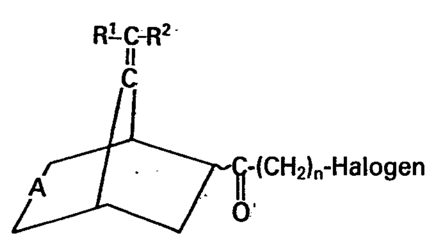

V

dans laquelle A, $R^1$ et $R^2$ ont les significations indiquées pour la formule I, n représente 0 ou 1 et halogène représente le brome ou le chlore, avec l'imidazole ou un sel d'imidazole de formule IV pour obtenir un composé de formule I, dans laquelle A, $R^1$ et $R^2$ ont les significations indiquées pour la formule I, B est le groupe $\underset{\underset{O}{\|}}{C}$ et n est égal à 0 ou 1, et éventuellement on le réduit en un composé dans lequel B représente le groupe CHOH et si on le désire, on convertit les composés obtenus suivants a) ou b) avec un acide en les sels d'addition d'acides physiologiquement acceptables.

2. Procédé selon la revendication 1, caractérisé en ce que l'on prépare un composé de formule I dans laquelle $R^1$ et $R^2$ sont identiques ou différents et représentent un groupe phényle, 4-chlorophényle ou 4-fluorophényle, A représente une liaison simple, n représente 0 ou 1 et B représente une liaison simple ou le groupe CO.

3. Procédé selon la revendication 2 caractérisé en ce que B représente le groupe CO.

4. Procédé de préparation d'une préparation pharmaceutique contenant un composé de formule I selon la revendication 1 ou un sel physiologiquement acceptable d'un tel composé, caractérisé en ce qu'on l'amène à une forme d'administration appropriée par addition de substances auxiliaires.